# EUROPEAN PATENT SPECIFICATION

(11) **EP 0 437 258 B1**
(45) Date of publication and mention of the grant of the patent: **29.11.1995**
(21) Application number: 91100253.3
(22) Date of filing: 09.01.1991
(51) Int. Cl.: C07C 271/28, C07C 269/00

(54) **Method of manufacturing aromatic urethane**
Verfahren zur Herstellung aromatischer Urethane
Méthode pour la préparation d'uréthanes aromatiques

(30) Priority: 12.01.1990 JP 5635/90; 13.04.1990 JP 98443/90; 15.11.1990 JP 309372/90
(43) Date of publication of application: 17.07.1991
(73) Proprietor: NKK CORPORATION, Tokyo (JP)
(72) Inventor: Ikariya, Takao, c/o Patent & License Dept., Chiyoda-ku, Tokyo (JP); Itagaki, Masanori, c/o Patent & License Dept., Chiyoda-ku, Tokyo (JP); Mizuguchi, Masatsugu, c/o Patent & License Dept., Chiyoda-ku, Tokyo (JP); Hachiya, Tetsuo, c/o Patent & License Dept., Chiyoda-ku, Tokyo (JP); Hattori, Toshiyuki, c/o Patent & License Dept., Chiyoda-ku, Tokyo (JP); Nakamura, Tomomichi, c/o Patent & License Dept., Chiyoda-ku, Tokyo (JP)
(74) Representative: Sajda, Wolf E., Dipl.-Phys.

(56) References cited:
- DD-A- 158 900
- DE-A- 3 117 249
- US-A- 2 409 712
- US-A- 2 677 698
- US-A- 2 806 051
- Houben-Weyl, Methoden der Organischen Chemie, Bd. E4, S.178,179 und 343

## Description

The present invention relates to a method of manufacturing an aromatic urethane.

A large number of methods have been conventionally proposed as a method of manufacturing an aromatic urethane.

A first method is a known manufacturing method using a reaction between an aromatic isocyanate and an alcohol. In this method, however, highly toxic phosgene gas must be used in the manufacture of an isocyanate.

A second method uses a catalyst of a metal or a metal compound to react a nitrogen-containing aromatic compound with carbon monoxide in the presence of an organic compound containing a hydroxyl group. Known examples of this method are methods using an aromatic nitro compound as the nitrogen-containing compound which is a starting material (e.g., Published Examined Japanese Patent Application No. 43-23939 and Published Unexamined Japanese Patent Application Nos. 51-98240 and 54-22339) and methods using an aromatic amino compound as the same (e.g., Published Unexamined Japanese Patent Application Nos. 55-120551, 55-124750, and 59-172451). In the second method, a catalyst is used in the form of a solid or dissolved in a reaction solution. In this method, however, even if a solid catalyst is used, a portion of the catalyst elutes into a reaction solution. In this method, therefore, the catalyst must be separated from a reaction system after the reaction, resulting in a cumbersome operation and a high manufacturing cost. In addition, since the produced aromatic urethane must be purified by distillation, a reduction in yield caused by decomposition upon purification cannot be avoided.

In a third method, a catalyst of a metal or a metal compound is used to react a nitrogen-containing aromatic compound with carbon monoxide, thereby synthesizing a substituted urea compound. Thereafter, the substituted urea compound is reacted with an organic compound containing a hydroxyl group to obtain an aromatic urethane. Examples of the third method are disclosed in Published Unexamined Japanese Patent Application Nos. 62-59251, 6-59252, and 62-111958 and US-A 4,678,856. Also in this method, since the metal catalyst is used in the first reaction, an increase in installation cost for separating and recovering the catalyst component from a reaction system cannot be avoided. In addition, since all of the above methods use highly toxic carbon monoxide and high-pressure reaction system, increases in installation cost and operation cost cannot be avoided.

In consideration of this, a method not using carbon monoxide has been proposed as a fourth method. For example, Published Unexamined Japanese Patent Application No. 56-103152 discloses a method of directly mixing and reacting urea, a primary amine, and an alcohol. In this method, however, since production of an alkyl-carbamate caused by a reaction between urea and an alcohol and production of aromatic urea caused by a reaction between urea and an aromatic amine simultaneously occur, the selectivity of an urethane compound cannot be increased. In this case, although a production rate of the alkyl-carbamate (NH₂COOR) is high because urea reacts with an alcohol, a production rate of an N-phenylcarbamate is low because the alkyl-carbamate hardly reacts with the aromatic primary amine. In addition, since an alcohol as a low boiling point compound is used, a high-pressure reactor vessel must be used, resulting in an increase in installation cost. Alternatively, as described in Published Examined Japanese Patent Application No. 63-42616, an aromatic urethane can be manufactured by reacting an aromatic amine with an O-alkylcarbamate at a normal pressure in the presence of an alcohol and urea. In this reaction, although no carbon monoxide having high toxicity is used, a catalyst such as a Lewis acid must be used to increase a reaction rate. In addition, since an alkyl carbamate must be used in an excessive amount to aniline, an aromatic urethane as a product and the alkylcarbamate are inevitably decomposed on heating even 100°C when the aromatic urethane is separated by distillation. In addition, since an alcohol as a low-boiling compound is used, a high-pressure reactor vessel must be used at a reaction temperature, resulting in an increase in installation cost.

Furthermore, a method in which an N,N'-disubstituted urea as an intermediate in the third method described above is manufactured first and then an aromatic urethane is manufactured by using the N,N'-disubstituted urea and an alcohol may be available. A known example of a method of manufacturing the N,N'-disubstituted urea is a method in which a reaction between an aromatic primary amine and urea is performed such that the aromatic amine is used in a molar excessive amount of 2.2 - 3.2 with respect to the urea, thereby producing the N,N'-disubstituted urea at a yield of about 70% (Journal of American Chemical Society, Vol. 44, P. 2,595, 1922). Although the reaction is simple in this method, production of monosubstituted urea cannot be avoided since, e.g. the reaction temperature is low, resulting in a low yield.

US-A 2,409,712 teaches that N,N'-disubstituted ureas reacts with alcohol, producing alkyl carbamate. However the U.S. Patent does not suggest that aromatic carbamate is produced from aromatic primary amine.

As described above, all conventional methods of manufacturing an aromatic urethane require a very cumbersome operation and a very high manufacturing cost.

It is a first object of the present invention to provide a method of industrially manufacturing an aromatic urethane, in which a reaction is performed without using highly toxic carbon monoxide at a high pressure nor a halogen compound, thereby solving various problems caused by the use of same.

It is a second object of the present invention to provide a method of manufacturing an aromatic urethane having increased yield.

The present invention is a method of manufacturing an aromatic urethane as defined in claim 1.

In the first step, urea is reacted with an aromatic primary amine to give an N,N'-disubstituted urea. This reaction is assumed to progress step-wise in accordance with the following reaction formula: 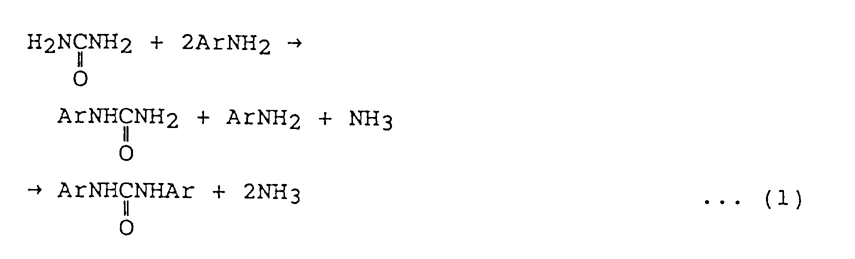
Examples of the aromatic primary amine used are an aniline, an aminonaphthalene, an aminoanthracene, and an aminoviphenyl. Examples of the compound are aniline, o-, m-, and p-toluidines, o-, m-, and p-chloroanilines, Ca- and Cb-naphthylamines, 2-methyl-1-aminonaphthalene, a diaminobenzene isomer, a triaminobenzene isomer, an aminotoluene isomer, a diaminotoluene isomer, an aminonaphthalin isomer, and mixtures of these compounds.

A reaction temperature is normally 50°C to 300°C, and preferably, 130°C to 250°C. A reaction pressure is a vapor pressure of a solvent at the reaction temperature. If the vapor pressure is lower than the atmospheric pressure, the reaction can be conducted under reduced pressure. A reaction time is normally several minutes to several hours though it depends on other conditions.

Since this reaction is an equilibrium reaction, removal of ammonia as a product out of the system is essential to realize a smooth reaction. It is effective to remove ammonia at a normal pressure under reflux of a solvent or to remove it by flowing an inert gas, e.g., nitrogen gas.

Although this reaction hardly causes a side reaction, an N-monosubstituted urea may be produced as a reaction intermediate. If this reaction intermediate remains unremoved, it is converted into an alkyl carbamate as an undesired by-product in the second step. In order to increase the yield of the entire process, therefore, the amounts of urea and the remaining N-monosubstituted urea must be minimized in performed in a batch-wise manner because the removal of ammonia can be perfectly performed and the reaction can be easily completed when the step is performed in a batch-wise manner.

In the method of the present invention, an aromatic primary amine as a material is also used as the reaction solvent. As shown in formula (1), a stoichiometric amount of the aromatic primary amine with respect to one mol of urea is two mols. Since the reaction is a reversible reaction, however, the concentration of the aromatic amine is preferably high in order to increase the reaction rate and to complete the reaction. Therefore, the aromatic primary amine is used in an equivalent amount or more with respect to urea. In the second step to be described later, the reaction rate and the conversion rate are increased as the concentration of the aromatic primary amine is decreased. It is, therefore, necessary to perform the first step by properly setting the concentration of the aromatic primary amine, the reaction temperature, and the reaction time so that 0.05 mol or less of urea, 0.05 mol or less of the N-monosubstituted urea, and 3.0 mol or less of the aromatic primary amine are present with respect to 1.0 mol of the N,N'-disubstituted urea in the product composition. Selection of the concentration of the aromatic primary amine, the reaction temperature, and the reaction time for obtaining the above composition range can be easily performed by those skilled in the art. That is, since urea and the N-monosubstituted urea are intermediates of the reaction, the reaction can be satisfactorily completed by (1) increasing the reaction temperature to increase the reaction rate, (2) setting a satisfactory reaction time, and (3) removing produced ammonia by the batch-wise method, thereby obtaining the predetermined composition range. In addition, since the aromatic primary amine is a material of the reaction, its consumption amount during the reaction can be calculated beforehand. By setting an amount at the start of the reaction to be a total of an amount within the above predetermined composition range and the consumption amount, therefore, an amount obtained when the reaction is completed can be a value within the predetermined composition range.

By setting the composition range as described above, the reaction solution in the first step is directly used in the second step without separation/purification step. As a result, a manufacturing cost can be largely reduced.

When a large amount of aromatic primary amine is used to attain higher reaction rate, the aromatic primary amine is preferably partially removed and then used in the second step so that 0.05 mol or less of urea, 0.05 mol or less of the N-monosubstituted urea, and 3.0 mol or less of the aromatic primary amine are present with respect to 1.0 mol of the N,N'-disubstituted urea in the product composition after the reaction.

The present inventors conducted experiments by changing the product compositions and found that the reaction rate, the yield and selectivity can be increased to increase the efficiency of the entire process when 0.05 mol or less of urea, 0.05 mol or less of an N-monosubstituted urea, and 3.0 mol or less of an aromatic primary amine are present with respect to 1.0 mol of an N,N'-disubstituted urea as the product composition, and that no satisfactory effect can be obtained when the production composition falls outside this range, thereby determining the above composition range.

Ammonia produced upon reaction in the first step is removed out of the reaction system in order to increase the reaction rate and the yield. For this reason, stripping using an inert gas such as nitrogen or argon is preferably used not only at a normal-pressure reaction but also in a reaction under pressure.

Ammonia by-produced in the reaction can be arbitrarily treated and it can be recovered outside a reactor and returned to the urea manufacturing process as feed stock if necessary.

In the first step, a catalyst need not particularly be used since a satisfactorily high reaction rate can be obtained without using a catalyst. A high reaction rate, however, can be obtained by the use of a catalyst. Preferable examples of the catalyst are a Lewis acid such as aluminum chloride, zinc chloride, iron(II) chloride, iron(III) chloride, tin(II) chloride, tin(IV) chloride, titanium(IV) chloride, antimony(V) chloride, aluminum bromide, boron trifluoride, boron trichloride, and boron tribromide; a metal alkoxide such as sodium methoxide, sodium ethoxide, lithium methoxide, and lithium ethoxide; and a Lewis base such as triethylamine and tri-n-propylamine.

In the second step, the obtained N,N'-disubstituted urea is reacted with an organic compound having a hydroxyl group (alcohol) as represented by the reaction formula, thereby producing an aromatic primary amine and an aromatic urethane: 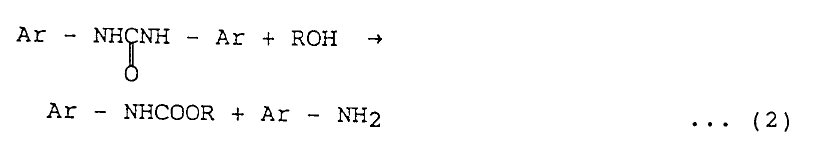
Since this reaction is also a reversible reaction, the reaction rate and the conversion rate can be increased as the concentration of the aromatic primary amine as a reaction product is decreased. Since the amounts of urea, the N-monosubstituted urea, and the aromatic primary amine are set to fall within a predetermined composition range in the first step, a compound having a hydroxyl group is directly added thereto. Examples of the organic compound having a hydroxyl group are a monovalent alcohol and a monovalent phenol. More specifically, examples are a monovalent alcohol such as methyl, ethyl, n-propyl, isopropyl, n-butyl, isobutyl, and t-butyl; and an alkylphenol having an alkyl group such as phenol, chlorophenol, methyl, ethyl, n-propyl, and isopropyl.

An alcohol is preferably used in an amount of 3.0 mol or more with respect to 1.0 mol of the N,N'-diphenyl urea as a material of the second step regardless of the first step. This is because the reaction rate and the conversion rate can be increased by increasing the concentration of the alcohol as a material since the reaction in the second step is also an equilibrium reaction.

The reaction temperature is normally 80°C to 300°C, and preferably, 130°C to 250°C. The reaction pressure is set at a vapor pressure at the reaction temperature of the organic compound containing a hydroxyl group or a solvent normally used. Even when a pressurized reaction vessel is required, the reaction can be performed in a batch-wise manner or continuously. In the continuous reaction, a tubular reactor can also be used in order to sufficiently complete the reaction. As a result, an installation cost can be reduced. This reaction can also be performed without using a catalyst.

When the reaction is completed, distillation is performed to recover an aromatic urethane as a residue. In addition, the aromatic primary amine and a solvent not concerning the reaction, if the solvent is used, are also recovered by the distillation.

In the third step, this aromatic primary amine or the aromatic primary amine containing the solvent not concerning the reaction is reused as a part of the material used in the production reaction of the N,N'-disubstituted urea in the first step.

By performing the first step (reaction formula (1)), the second step (reaction formula (2)), and the third step as described above, one molecule of an aromatic urethane and two molecules of ammonia are synthesized from one molecule of urea, one molecule of aniline, and one molecule of the organic solvent containing a hydroxyl group: 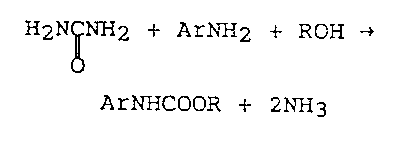
When no catalyst is used in the reaction in either the first or second step, a complicated operation for separating and recovering a catalyst from the reaction system and an installation cost therefor are not required and no high installation cost is necessary since the first reaction does not require a high-pressure apparatus. In addition, since no halogen compound is used, a problem of corrosion of the material of an apparatus does not arise.

Since the reaction is performed in the first step such that 0.05 mol or less of urea, 0.05 mol or less of an N-monosubstituted urea, and 3.0 mol or less of an aromatic primary amine are present with respect to 1.0 mol of an N,N'-disubstituted urea as the product composition, this reaction product can be directly supplied to the second step without performing a separation/purification step. In addition, even when a large amount of aromatic primary amine is used, the aromatic primary amine need only be partially removed, and a crystallization/filtering step requiring a cumbersome operation and a high cost need not be performed. In this manner, an aromatic urethane can be manufactured at satisfactorily high reaction and conversion rates by sequentially performing the first and second steps.

In addition, the conversion is further increased by performing the first reaction step in a batch-wise manner.

In the second reaction, the selectivity from the N,N-disubstituted urea to the aromatic urethane is very high, and an amount of a by-product is very small. In the method of the present invention adopting the two reaction steps, therefore, an aromatic urethane can be manufactured at high yield from an aromatic amine and an organic compound having a hydroxyl group.

Since aromatic urethane produced by the present invention is unstable at high temperature, it should not be heated during the purifying step of aromatic urethane. The solvent extraction is preferably applied to purify the aromatic urethane produced by the method of the present invention. Since the aromatic urethane has a small amount of impurities, the solvent extraction is more advantageous for purifying the aromatic urethane

In addition, by-produced ammonia can be trapped as a pure product and reused in the urea manufacturing process as needed.

The present invention will be described in more detail below by way of its examples.

### Example 1 (a product obtained in the 1st step is directly used in the 2nd step)

401 mmol of urea and 1,205 mmol of aniline were charged in a four-necked flask having an internal volume of 500 mℓ with a gas inlet cock and a reflux condenser having an exhaust bubbler mounted on its upper portion in an N₂ atmosphere, heated on an oil bath, and reacted under reflux at normal pressure for three hours. The reaction temperature was 186°C. The releasing ammonia gas was stopped, when the reaction was completed, the reaction solution was cooled to room temperature, and the contents in the flask were analyzed by a liquid chromatography. As a result, 391 mmol of N,N'-diphenyl urea (to be abbreviated to as DPU hereinafter) and 10 mmol of N-phenyl urea (to be abbreviated to as MPU hereinafter) were present, but urea as a material was not detected. The yield of the DPU with respect to urea used was 98%.

The entire contents in the flask and 184.73 g of ethanol were charged in a stainless steel electromagnetically stirred autoclave having an internal volume of 500 mℓ and reacted under stirring at 175°C for two hours. When the reaction was completed, the reaction solution was analyzed by a gas chromatography. As a result, 371 mmol of N-ethylphenylcarbamate and 751 mmol of aniline were present. The yield of N-ethylphenylcarbamate as a whole was 93%.

### Example 2 (a product obtained in the 1st step is used in the 2nd step after aniline is partially removed from the product)

25.0 mmol of urea and 547.6 mmol of aniline were charged in a four-necked flask having an internal volume of 500 mℓ with a gas inlet cock and a reflux condenser having an exhaust bubbler mounted on its upper portion in an argon atmosphere, heated under stirring on an oil bath, and reacted under reflux at normal pressure for 40 minutes. In this reaction, generation of ammonia gas was confirmed. When the reaction was completed, the reaction solution was cooled to room temperature and filtered to obtain 4.97 g (23.4 mmol) of DPU crystals. When the filtrate was analyzed by a HPLC, 0.17 g (0.8 mmol) of DPU were contained, but no MPU was detected. The yield of the DPU with respect to urea was 97%.

Aniline was partially removed from the reaction mixture by distillation, and the resultant composition was analyzed. As a result, 24.2 mmol of DPU and 51.0 mmol of aniline were present. This mixture and 50 g of ethanol were charged in a stainless steel electromagnetically stirred autoclave having an internal volume of 200 mℓ and reacted under stirring at 175°C for three hours. When the reaction was completed, the reaction solution was analyzed by a gas chromatography. As a result, the yield of N-ethylphenylcarbamate was 94%, and that of aniline was 94%.

### Examples 3-6

In order to check the effect of a catalyst, 2.0 g of urea 100 g of aniline, and a material listed in Table 1 (to be presented later) were charged in a four-necked flask having an internal volume of 200 mℓ with a reflux condenser having an exhaust bubbler mounted on its upper portion in an N₂ atmosphere, heated under stirring on an oil bath, and reacted under reflux at a normal pressure for one hour. The reacted temperature was adjusted to be 158°C. When the reaction was completed, DPU in the reaction solution was analyzed by a liquid chromatography. The results are summarized in Table 1.

### Comparative Example 1

0.50 g of urea and 50 mℓ of aniline were charged in an autoclave having an internal volume of 100 mℓ with an electromagnetic stirrer in an argon atmosphere and reacted at 160°C for two hours. In this reaction, the pressure in the reactor was a spontaneous pressure of aniline. The reaction solution was cooled to room temperature and filtered to obtain 0.60 g of DPU crystals. When the filtrate was analyzed, 0.02 g of DPU, 0.45 g of NPU, and 0.02 g of nonreacted urea were detected. The yield of DPU was 35% and that of NPU was 40% with respect to urea.

### Comparative Example 2

In accordance with a method described in "Organic Synthesis", Coll., Vol. I, P. 442, 3.9 g of aniline hydrochloride and 1.9 g of urea were reacted in a flask with a reflux condenser under boiling at 100°C for one hour. A precipitated solid mixture was filtered under suction and heating to obtain 1.5 g of DPU as a crude product. The yield was 22% with respect to urea.

### Comparative Example 3

333 mmol of urea, 600 mmol of aniline, and 1,623 mmol of ethanol were charged in a stainless steel autoclave having an internal volume of 300 mℓ, and nitrogen was flowed under bubbling at a flow rate of 400 mℓ/min. The internal pressure of the autoclave was set at 16 kg/cm² by a dwell valve. The reaction was performed under stirring by an electromagnetic stirrer at 180°C for two hours. When the reaction was completed, the reaction solution was analyzed by a gas chromatography. As a result, 122 mmol of N-ethylphenylcarbamate and 417 mmol of aniline were present. In addition, 30.5 mmol of unreacted urea, 33.4 mmol of MPU, 15.1 mmol of DPU, and 126 mmol of nonsubstituted ethylcarbamate were present. The conversion rate of urea was 90.8%, and the selectivity of N-ethylphenylcarbamate was 41.4%.

### Controls 1 & 2 (a reaction in the 2nd step obtained when a product composition in the 1st step falls outside a predetermined range)

Following the same procedures as in the second step of the method of the present invention, predetermined amounts of materials were charged in an electromagnetically stirred autoclave having an internal volume of 200 mℓ and reacted at a temperature of 175°C and a spontaneous pressure for three hours. The experimental results are summarized in Table 2.

The experimental results reveal that when the amount of urea and N-monosubstituted urea fall outside the defined composition range of the present invention, a large amount of nonsubstituted ethylcarbamate as an undesired by-product was produced. In addition, when the amount of aniline falls outside the composition range of the present invention, nonreacted urea, N-monosubstituted urea, and N,N'-diphenyl urea undesirably remain.

**Table 1**

| Example | Catalyst | Yield (%) |
|---|---|---|
| 3 | No | 53 |
| 4 | Aluminum Chloride 0.446 g | 60 |
| 5 | Zinc Chloride 0.481 g | 75 |
| 6 | Sodium Methoxide 0.181 g | 56 |

**Table 2**

| | | Control 1 | Control 2 |
|---|---|---|---|
| charge (mmol) | Urea | 33* | 33* |
| | MPU | 66* | 66* |
| | DPU | 100 | 100 |
| | Aniline | 0 | 110 |
| | Ethanol | 1003 | 784 |
| After Reaction (mmol) | Urea | 6 | 6 |
| | MPU | 14 | 20 |
| | DPU | 7 | 14 |
| | Aniline | 116 | 219 |
| | EC | 40 | 39 |
| | EPC | 138 | 135 |
| | EPC Yield (%) | 69 | 68 |

| | | | |
|---|---|---|---|
| * Outside designated range | | | |
| EC: Nonsubstituted ethylcarbamate EPC: N-ethylphenylcarbamate An EPC yield was calculated with reference to (urea + MPU + DPU). | | | |

## Claims

1. A method of manufacturing an aromatic urethane by using an aromatic primary amine, urea, and an alcohol as materials, said method comprising:
a first step of performing a reaction between the aromatic primary amine and urea while removing ammonia produced in the reaction to produce an N,N'-disubstituted urea;
a second step of reacting an alcohol with the reaction product obtained from said first step; and
a third step of separating the reaction products produced in said second step from each other;
**characterized in that**
said first step is performed in batch-wise manner by adjusting the composition of the materials such that not more than 0.05 mol of urea, not more than 0.05 mol of an N-monosubstituted urea, and not more than 3.0 mol of the aromatic primary amine are present with respect to 1.0 mol of the N,N'-disubstituted urea in a product composition after the reaction;
said second step is performed by directly using the product obtained in said first step without purifying the product and is performed to produce aromatic urethane and aromatic primary amine;
said third step comprises separating the aromatic urethane and the aromatic primary amine and circulating the aromatic primary amine to said first step.

2. A method according to claim 1, characterized in that said first step includes a step of partially removing the aromatic primary amine.

3. A method according to claim 1, characterized in that said first step is performed in the presence of or in the absence of a catalyst.

4. A method according to claim 1, characterized in that said second step is performed in the presence of or in the absence of a catalyst.

5. A method according to claim 3, characterized in that in said first step is performed in the presence of a catalyst selected from a Lewis acid selected from the group consisting of aluminum chloride, zinc chloride, iron (II) chloride, iron (III) chloride, tin (II) chloride, tin (IV) chloride, titanium(IV) chloride, antimony(V) chloride, aluminum bromide, boron trifluoride, boron trichloride, and boron tribromide; a metal alkoxide selected from the group consisting of sodium methoxide, sodium ethoxide, lithium methoxide, and lithium ethoxide; a Lewis base selected from the group consisting of triethylamine and tri-n-propylamine; and mixtures thereof.

6. A method according to claim 5, characterized in that said catalyst is selected from the group consisting of a catalyst devoid of carrier, a solid catalyst solidified by a carrier, a solid acid catalyst solidified by a carrier, and a mixture thereof.

7. A method according to one of the claims 1 to 6, characterized in that said first and second steps are performed at a temperature of 130°C to 250°C.

8. A method according to one of the claims 1 to 7, characterized in that in said second step, not less than 3.0 mol of the alcohol are used with respect to 1.0 mol of an N,N'-diphenyl urea.

## Patentansprüche

1. Verfahren zur Herstellung eines aromatischen Urethans unter Verwendung von einem primären aromatischen Amin, Harnstoff und einem Alkohol als Umsetzungskomponenten, das folgendes aufweist:
einen ersten Schritt der Durchführung einer Umsetzung zwischen dem primären aromatischen Amin und Harnstoff unter Entfernung des während der Reaktion erzeugten Ammoniaks, um einen N,N'-disubstituierten Harnstoff zu erzeugen;
einen zweiten Schritt des Umsetzens eines Alkohols mit dem Reaktionsprodukt, das aus dem ersten Schritt erhalten wurde; und
einen dritten Schritt der Trennung der Reaktionsprodukte, die in dem zweiten Schritt erzeugt wurden, voneinander;
dadurch gekennzeichnet,
daß der erste Schritt chargenweise durch Einstellen der Zusammensetzung der Umsetzungskomponenten derart durchgeführt wird, daß nicht mehr als 0,05 Mol Harnstoff, nicht mehr als 0,05 Mol eines N-monosubstituierten Harnstoffs und nicht mehr als 3,0 Mol des primären aromatischen Amins, in Bezug auf 1,0 Mol des N,N'-disubstituierten Harnstoffs, nach der Reaktion in der Produktzusammensetzung vorhanden sind;
daß der zweite Schritt durch unmittelbare Verwendung des Produktes, das im ersten Schritt erhalten wurde, ohne Reinigung des Produktes durchgeführt, und zwar mit dem Ziel durchgeführt wird, aromatisches Urethan und primäres aromatisches Amin zu erzeugen;
und daß der dritte Schritt das Trennen des aromatischen Urethans und des primären aromatischen Amins und Rückführen des primären aromatischen Amins in den ersten Schritt aufweist.

2. Verfahren nach Anspruch 1,
dadurch gekennzeichnet,
daß der erste Schritt einen Schritt der teilweisen Entfernung des primären aromatischen Amins einschließt.

3. Verfahren nach Anspruch 1,
dadurch gekennzeichnet, daß der erste Schritt in Anwesenheit oder in Abwesenheit eines Katalysators durchgeführt wird.

4. Verfahren nach Anspruch 1,
dadurch gekennzeichnet,
daß der zweite Schritt in Anwesenheit oder in Abwesenheit eines Katalysators durchgeführt wird.

5. Verfahren nach Anspruch 3,
dadurch gekennzeichnet,
daß der erste Schritt in Anwesenheit eines Katalysators durchgeführt wird, der aus einer Lewis-Säure, die ausgewählt aus der Gruppe, bestehend aus Aluminiumchlorid, Zinkchlorid, Eisen(II)chlorid, Eisen(III)chlorid, Zinn(II)chlorid, Zinn(IV)chlorid, Titan(IV)chlorid, Antimon(V)chlorid, Aluminiumbromid, Bortrifluorid, Bortrichlorid und Bortribromid; einem Metallalkoholat, das ausgewählt aus der Gruppe, bestehend aus Natriummethanolat, Natriumethanolat, Lithiummethanolat und Lithiumethanolat; einer Lewis-Base, die ausgewählt aus der Gruppe, bestehend aus Triethylamin und Tri-n-propylamin; und Gemischen daraus.

6. Verfahren nach Anspruch 5,
dadurch gekennzeichnet,
daß der Katalysator aus der Gruppe ausgewählt wird, die aus einem Katalysator ohne Trägersubstanz, einem festen Katalysator, der durch eine Trägersubstanz verfestigt wurde, einem festen Säurekatalysator, der durch eine Trägersubstanz verfestigt wurde, und Gemischen daraus besteht.

7. Verfahren nach einem der Ansprüche 1 bis 6,
dadurch gekennzeichnet,
daß der erste und der zweite Schritt bei einer Temperatur von 130 °C bis 250 °C durchgeführt werden.

8. Verfahren nach einem der Ansprüche 1 bis 7,
dadurch gekennzeichnet,
daß im zweiten Schritt nicht mehr als 3,0 Mol des Alkohols, in Bezug auf 1,0 Mol eines N,N'-Diphenylharnstoffs, verwendet werden.

## Revendications

1. Procédé de fabrication d'un uréthane aromatique en utilisant une amine primaire aromatique, de l'urée, et de l'alcool en tant que matériaux, ledit procédé comprenant :
une première étape consistant à effectuer une réaction entre l'amine primaire aromatique et l'urée tout en enlevant l'ammoniaque produit dans la réaction, pour produire une urée N, N' - disubstituée;
une seconde étape consistant à faire réagir' un alcool avec le produit de réaction obtenu de ladite première étape ; et
une troisième étape consistant à séparer les uns des autres les produits de réaction produits dans ladite seconde étape ;
caractérisé en ce que
ladite première étape est mise en oeuvre de manière discontinue en ajustant la composition des matériaux de telle manière que par rapport à 1,0 moles de l'urée N, N' - disubstituée dans la composition des produits après la réaction il n'existe pas plus de 0,05 moles d'urée, pas plus de 0,05 moles durée N - monosubstituée, et pas plus de 3,0 moles de l'amine primaire aromatique ;
ladite seconde étape est mise en oeuvre en utilisant directement le produit obtenu dans ladite première étape sans le purifier, et elle est mise en oeuvre pour produire de l'uréthane aromatique et l'amine primaire aromatique ;
ladite troisième étape comprend les opérations consistant à séparer l'uréthane aromatique et l'amine primaire aromatique et à faire circuler l'amine primaire aromatique vers ladite première étape.

2. Procédé selon la revendication 1, caractérisé en ce que ladite première étape comprend une étape consistant à enlever partiellement l'amine primaire aromatique.

3. Procédé selon la revendication 1, caractérisé en ce que ladite première étape est mise en oeuvre en présence ou en absence d'un catalyseur.

4. Procédé selon la revendication 1, caractérisé en ce que ladite seconde étape est mise en oeuvre en présence ou en absence d'un catalyseur.

5. Procédé selon la revendication 3, caractérisé en ce que ladite première étape est mise en oeuvre en présence d'un catalyseur choisi parmi un acide de Lewis choisi dans le groupe comprenant chlorure d'aluminium, chlorure de zinc, chlorure de fer (II), chlorure de fer (III), chlorure d'étain (II), chlorure d'étain (IV), chlorure de titane (IV), chlorure d'antimoine (V), bromure d'aluminium, trifluorure de bore, trichlorure de bore, et tribromure de bore ; un alkoxyde métallique choisi dans le groupe comprenant sodium méthoxyde, sodium éthoxyde, lithium méthoxyde, et lithium éthoxyde ; une base de Lewis choisie parmi le groupe comprenant la triéthylamine et la tri-n-propylamine ; et les mélanges de ceux-ci.

6. Procédé selon la revendication 5, caractérisé en ce que ledit catalyseur est choisi dans le groupe comprenant un catalyseur dépourvu de support, un catalyseur solide solidifié par un support, un catalyseur acide solide solidifié par un support, et un mélange de ceux-ci.

7. Procédé selon l'une quelconque des revendications 1 à 6, caractérisé en ce que ladite première étape et ladite seconde étape sont mises en oeuvre à une température de 130° C à 250° C.

8. Procédé selon l'une quelconque des revendications 1 à 7, caractérisé en ce que dans ladite seconde étape, on utilise par rapport à 1,0 moles d'une N, N' - diphényl urée une quantité d'alcool qui n'est pas inférieure à 3,0 moles.
